(19) Europäisches Patentamt / European Patent Office / Office européen des brevets

(11) **EP 3 483 281 A1**

(12) **EUROPEAN PATENT APPLICATION**
published in accordance with Art. 153(4) EPC

(43) Date of publication:
**15.05.2019 Bulletin 2019/20**

(21) Application number: **18814244.2**

(22) Date of filing: **28.05.2018**

(51) Int Cl.:
*C12P 19/04* (2006.01)    *A23K 20/163* (2016.01)
*A23L 33/125* (2016.01)    *A61K 8/73* (2006.01)
*A61K 31/715* (2006.01)    *A61P 37/08* (2006.01)
*C08B 37/00* (2006.01)    *C12P 19/28* (2006.01)
*B01D 15/36* (2006.01)    *C12N 1/20* (2006.01)

(86) International application number:
**PCT/JP2018/020334**

(87) International publication number:
**WO 2018/225557 (13.12.2018 Gazette 2018/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.06.2017 JP 2017114172**

(71) Applicant: **Asahi Kohsan Corporation
Tokyo 160-0022 (JP)**

(72) Inventor: **SUGIYAMA,Masanori
Hiroshima-shi
Hiroshima 732-0063 (JP)**

(74) Representative: **Papula Oy
P.O. Box 981
00101 Helsinki (FI)**

(54) **EXTRACELLULAR POLYSACCHARIDE OF LACTIC ACID BACTERIA AND USE THEREOF**

(57) An exopolysaccharide of a lactic acid bacterium derived from a fig and belonging to *Lactobacillus paracasei* includes a neutral polysaccharide having a structure in which N-acetylglucosamines are linked with each other via α-1,6 bond, has a hyaluronidase inhibitory, and is therefore useful in a food and drink, a medicine, a feed, a cosmetic and the like exerting an antiallergy effect.

FIG.1

(A)

(B)

NL  D3.9  x12k  5.0 μm

SEQUENCE LISTING
<110> Asahi Kohsan Corporation
<120> Exopolysaccharide of Lactic Acid Bacteria and Use Thereof
<150> JP 2017-114172
<151> 2017.6.9
<160> 3
<170> PatentIn version 3.1.2


<210> 1
<211> 20
<212> DNA
<213> Artificial sequence

<400> 1
agagtttgat cctggctcag                                                     20


<210> 2
<211> 18
<212> DNA
<213> Artificial sequence

<400> 2
aaaggaggtg atccagcc                                                       18


<210> 3
<211> 1549
<212> DNA
<213> Lactobacillus sp.

<400> 3
atttatatga gagtttgatc ctggctcagg atgaacgctg gcggcgtgcc taatacatgc\201@   60
aagtcgaacg agttctcgtt gatgatcggt gcttgcaccg agattcaaca tggaacgagt\201@  120
ggcggacggg tgagtaacac gtgggtaacc tgcccttaag tggggataa catttggaaa\201@  180
cagatgctaa taccgcatag atccaagaac cgcatggttc ttggctgaaa gatggcgtaa\201@  240
gctatcgctt ttggatggac ccgcggcgta ttagctagtt ggtgaggtaa tggctcacca\201@  300
aggcgatgat acgtagccga actgagaggt tgatcggcca cattgggact gagacacggc\201@  360
ccaaactcct acgggaggca gcagtaggga atcttccaca atggacgcaa gtctgatgga\201@  420
gcaacgccgc gtgagtgaag aaggctttcg ggtcgtaaaa ctctgttgtt ggagaagaat\201@  480
ggtcggcaga gtaactgttg tcggcgtgac ggtatccaac cagaaagcca cggctaacta\201@  540
cgtgccagca gccgcggtaa tacgtaggtg gcaagcgtta tccggattta ttgggcgtaa\201@  600
agcgagcgca ggcggttttt taagtctgat gtgaaagccc tcggcttaac cgaggaagcg\201@  660
catcggaaac tgggaaactt gagtgcagaa gaggacagtg gaactccatg tgtagcggtg\201@  720
aaatgcgtag atatatggaa gaacaccagt ggcgaaggcg gctgtctggt ctgtaactga\201@  780
cgctgaggct cgaaagcatg ggtagcgaac aggattagat accctggtag tccatgccgt\201@  840
aaacgatgaa tgctaggtgt tggagggttt ccgcccttca gtgccgcagc taacgcatta\201@  900
agcattccgc ctggggagta cgaccgcaag gttgaaactc aaaggaattg acgggggccc\201@  960
gcacaagcgg tggagcatgt ggtttaattc gaagcaacgc gaagaacctt accaggtctt\201@1020
gacatctttt gatcacctga gagatcaggt ttcccccttcg ggggcaaaat gacaggtggt\201@1080
gcatggttgt cgtcagctcg tgtcgtgaga tgttgggtta agtcccgcaa cgagcgcaac\201@1140
ccttatgact agttgccagc atttagttgg gcactctagt aagactgccg gtgacaaacc\201@1200
ggaggaaggt ggggatgacg tcaaatcatc atgccccttca tgacctgggc tacacacgtg\201@1260
ctacaatgga tggtacaacg agttgcgaga ccgcgaggtc aagctaatct cttaaagcca\201@1320
ttctcagttc ggactgtagg ctgcaactcg cctacacgaa gtcggaatcg ctagtaatcg\201@1380
cggatcagca cgccgcggtg aatacgttcc cgggccttgt acacaccgcc cgtcacacca\201@1440
tgagagtttg taacacccga agccggtggc gtaacccttt agggagcga gccgtctaag\201@1500
gtgggacaaa tgattagggt gaagtcgtaa caaggtagcc gtaggagaa          \201@
1549

**Description**

Technical Field

The present invention relates to an exopolysaccharide of a lactic acid bacterium and the use thereof. More specifically, the present invention relates to an exopolysaccharide of a lactic acid bacterium that is derived from a fig and belongs to *Lactobacillus paracasei,* and also relates to a composition, such as a food and drink composition, a pharmaceutical composition, a feed composition and a cosmetic composition, comprising the exopolysaccharide and exerting an antiallergy effect and the like.

[Background Art]

Lactic acid bacteria are a group of bacteria that ferment carbohydrates such as glucose to acquire energy and produce a large amount of lactic acid and are nonpathogenic and non-spore-forming gram-positive bacteria. Lactic acid bacteria have been used for the preparation of fermented foods such as yogurt and cheese for a long time and are widely used as probiotics because they exert a beneficial effect for the health care of hosts when administered at an appropriate dose.

For example, *Lactobacillus plantarum* strain MA2 (Non-Patent Document 1) having an effect on serum lipid, *Lactobacillus plantarum* strain PH04 having an action of reducing cholesterol (Non-Patent Document 2), and the like are known as lactic acid bacteria as probiotics. In addition, Pediococcus pentosaceus strain LP28 (Non-Patent Document 3) having an effect of improving fatty liver and suppressing accumulation of fat in the body, and the like are known as plant-derived lactic acid bacteria.

*Lactobacillus paracasei* strain K71 having an antiallergy action (Patent Document 1), *Lactobacillus paracasei* strain MCC1375 having an anti-influenza virus activity (Patent Document 2), *Lactobacillus paracasei* strain KW3110 activating interleukin-12 production (Non-Patent Document 4), and the like are also known as lactic acid bacteria strains belonging to *Lactobacillus paracasei.*

On the other hand, with regard to exopolysaccharides produced by lactic acid bacteria, it is known that lactic acid bacteria-producing exopolysaccharides are used as starter strain of yogurt, to increase the viscosity of yogurt to prevent separation and generate a mellow texture specific for yogurt. Thus, lactic acid bacteria producing exopolysaccharides have been widely used in foods. In addition, exopolysaccharides may also be used as a thickener for foods. Furthermore, some lactic acid bacterial strains are known to produce exopolysaccharides as physiologically active substances that contribute to the maintenance and improvement of human health. It has been shown that exopolysaccharides produced by lactic acid bacteria exert an immunomodulatory function and an anti-gastritis effect (Non-Patent Documents 5 and 6). In addition, exopolysaccharides produced by lactic acid bacteria belonging to *Lactobacillus paracasei* are also known, and it has been reported that *Lactobacillus paracasei* strain DG produces rhamnose-rich exopolysaccharides (Non-Patent Document 7), and that *Lactobacillus paracasei* strain KB28 produces glucose-rich exopolysaccharides (Non-Patent Document 8). It has also been reported that *Lactobacillus paracasei* strain 34-1 produces an exopolysaccharide composed of D-galactose, 2-acetamido-2-deoxy-D-galactose and sn-glycerol 3-phosphate (Non-Patent Document 9).

Prior Art Documents

Patent Documents

    Patent Document 1: WO2009/131208
    Patent Document 2: WO2012/133827
    Non-Patent Documents

    Non-Patent Document 1: Appl. Microbiol. Biotechnol., 84, 341-347 (2009)
    Non-Patent Document 2: Int. J. Food Microbiol., 113, 358-361 (2007)
    Non-Patent Document 3: PLoS One e30696 (2012)
    Non-Patent Document 4: Biosci. Biotechnol. Biochem., 73, 1561-1565
    Non-Patent Document 5: Carbohydr. Polym., 124, 292-301 (2015)
    Non-Patent Document 6: Biol. Pharm. Bull., 17, 1012-1017 (1994)

Non-Patent Document 7: Appl. Environ. Microbiol., 83, e02702-16 (2017)
Non-Patent Document 8: J. Microbiol. Biotechnol., 21, 1174-1178 (2011)
Non-Patent Document 9: Carbohdr. Res., 285, 129-139 (1996)

Summary of Invention

Problem to be Solved by Invention

In light of the background art, the development of a novel exopolysaccharide produced by a lactic acid bacterium has been desired. Therefore, the problem to be solved by the present invention is to provide a novel exopolysaccharide produced by a lactic acid bacterium, which is effective as an active ingredient of a composition such as a food and drink composition, a pharmaceutical composition, a feed composition and a cosmetic composition, and the use thereof.

Means for Solving the Problem

The inventor of the present invention, as a result of intensive studies for the purpose of developing a novel exopolysaccharide produced by a lactic acid bacterium, isolated and identified a novel lactic acid bacterium belonging to *Lactobacillus paracasei* from figs, and found that this novel lactic acid bacterium produces an exopolysaccharide having a hyaluronidase inhibitory activity and particularly produces a novel polysaccharide having a structure which had not been observed at all in conventional lactic acid bacteria and in which N-acetylglucosamines were linked with each other via $\alpha$-1,6 bond, and through further studies based on such findings, the present invention has been completed.

In one aspect of the present invention, the present invention relates to an exopolysaccharide of a lactic acid bacterium that is derived from figs and belongs to *Lactobacillus paracasei.*

The exopolysaccharide of the present invention is preferably a neutral polysaccharide having a structure in which N-acetylglucosamines are linked with each other via $\alpha$-1,6 bond, and the neutral polysaccharide has a hyaluronidase inhibitory activity.

In addition, the exopolysaccharide of the present invention is preferably an acidic polysaccharide composed mainly of glucose and mannose, and the acidic polysaccharide has a hyaluronidase inhibitory activity.

The lactic acid bacterium producing the exopolysaccharide of the present invention is preferably *Lactobacillus paracasei* strain IJH-SONE68 (Accession No. NITE BP-02242) or a lactic acid bacterium equivalent thereto.

The lactic acid bacterium producing the exopolysaccharide of the present invention is obtainable by isolating and purifying, by an ion exchange chromatography, polysaccharides obtained from the culture of a lactic acid bacterium that is derived from a fig and belongs to *Lactobacillus paracasei.* More specifically, the exopolysaccharide of the present invention is obtainable by the steps of (1) removing bacterial cell bodies by centrifugation from the culture of a lactic acid bacterium derived from a fig and belonging to *Lactobacillus paracasei;* (2) recovering polysaccharides and proteins as precipitates from the culture obtained in the step (1), by precipitation due to ethanol or acetone; (3) removing the proteins from the recovered precipitates to recover exopolysaccharides; and (4) isolating and purifying the recovered exopolysaccharides by an anion exchange chromatography.

In other aspect of the present invention, the present invention relates to a composition comprising the exopolysaccharide as described above.

The composition is preferably a food and drink composition, and the food and drink preferably include a beverage, a functional food, a fermented food and a supplement. In addition, the composition is preferably a pharmaceutical composition. The composition is also preferably a feed composition and a cosmetic composition.

These compositions are preferably used for a hyaluronidase inhibition or an antiallergy.

Effect of Invention

The exopolysaccharide produced by the lactic acid bacterium of the present invention has an activity of inhibiting hyaluronidase that is an enzyme hydrolyzing hyaluronic acid, and is therefore effective as a food and drink, a medicine, a feed, and a cosmetic product which each exert an antiallergy effect and the like.

Brief Description of Drawings

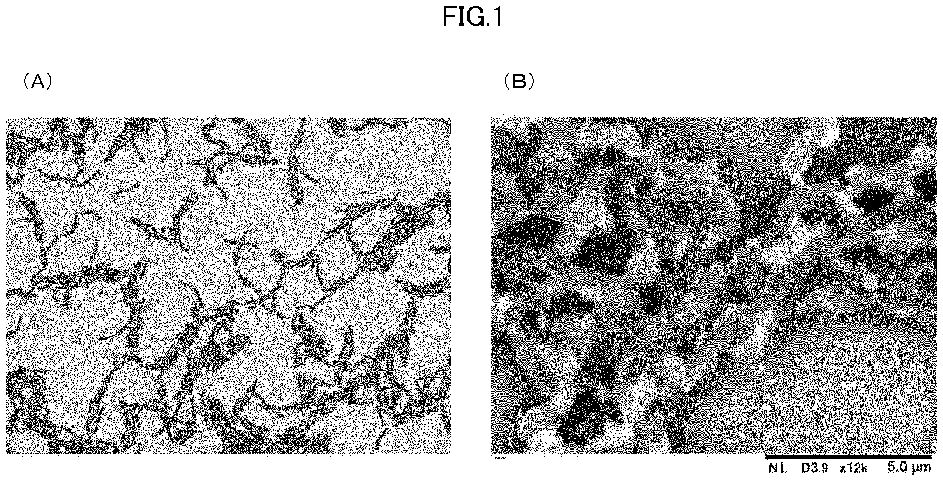

Fig. 1 is microscope photographs of *Lactobacillus paracasei* strain IJH-SONE68 isolated and identified according to the present invention. (A) in Fig. 1 is a gram-stained microscope photograph, and (B) in Fig. 1 is a scanning electron microscope (SEM) photograph.

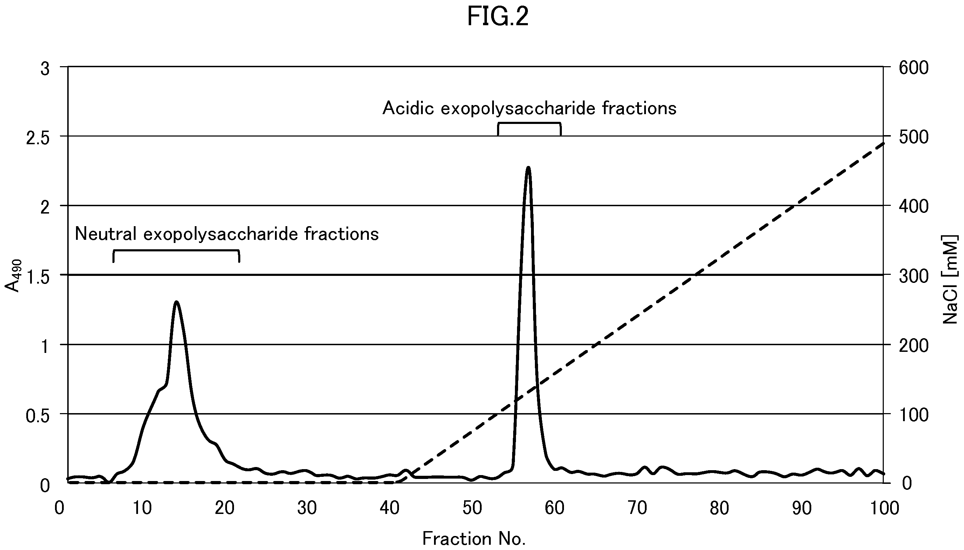

Fig. 2 illustrates an isolation profile of anion exchange chromatography (TOYOPEARL DEAE-650M resin (Tosoh Corporation)) of exopolysaccharides from *Lactobacillus paracasei* strain IJH-SONE68. The exopolysaccharides were eluted with NaCl having a gradient concentration of 0 mM to 500 mM (broken line), and the exopolysaccharide in each fraction was monitored at 490 nm by a phenol sulfuric acid method (straight line).

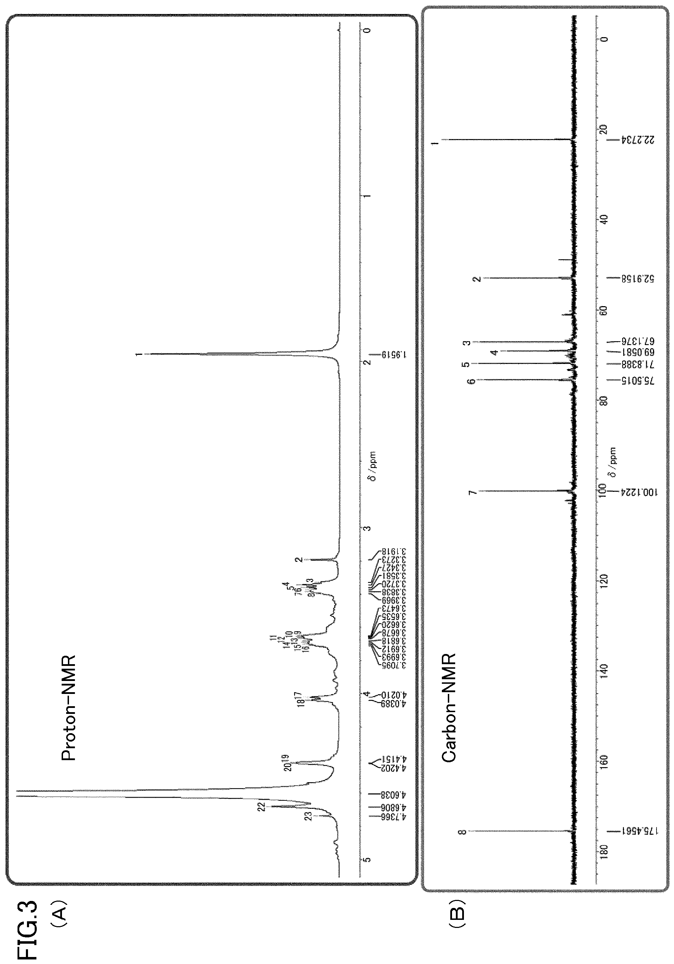

Fig. 3 illustrates each NMR profile obtained by subjecting a neutral exopolysaccharide, which was obtained by purifying exopolysaccharides from *Lactobacillus paracasei* strain IJH-SONE68 with anion exchange column chromatography, to proton-NMR and carbon-NMR. (A) in Fig. 3 is the NMR profile of proton-NMR, and (B) in Fig. 3 is the NMR profile of carbon-NMR.

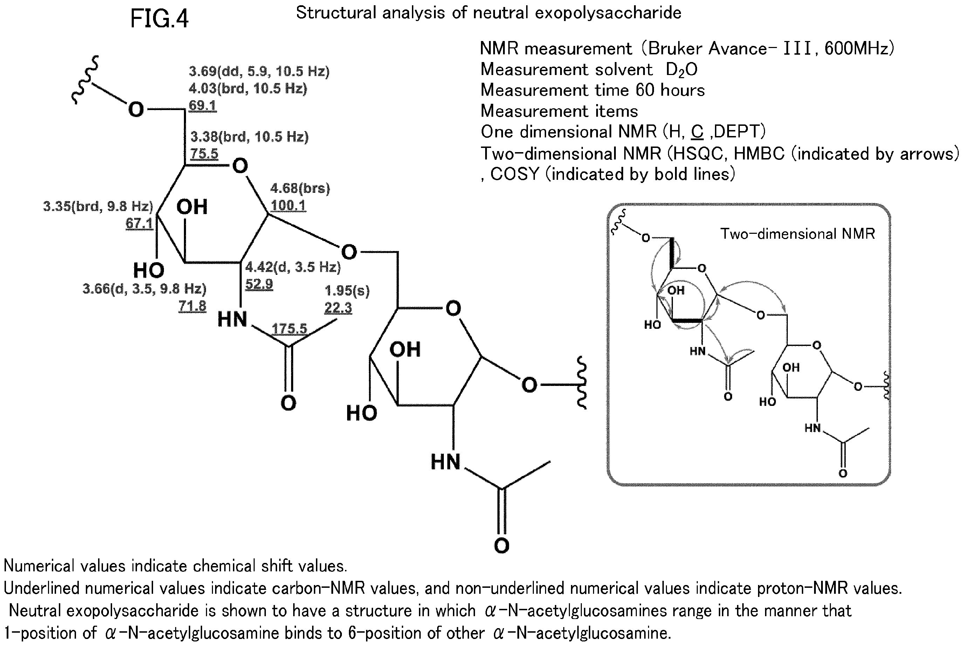

Fig. 4 illustrates results of structurally analyzing a neutral exopolysaccharide on the basis of the NMR profile. These structural analysis results revealed that the neutral exopolysaccharide of *Lactobacillus paracasei* strain IJH-SONE68 has a structure in which N-acetylglucosamines are linked with each other via $\alpha$-1,6 bond.

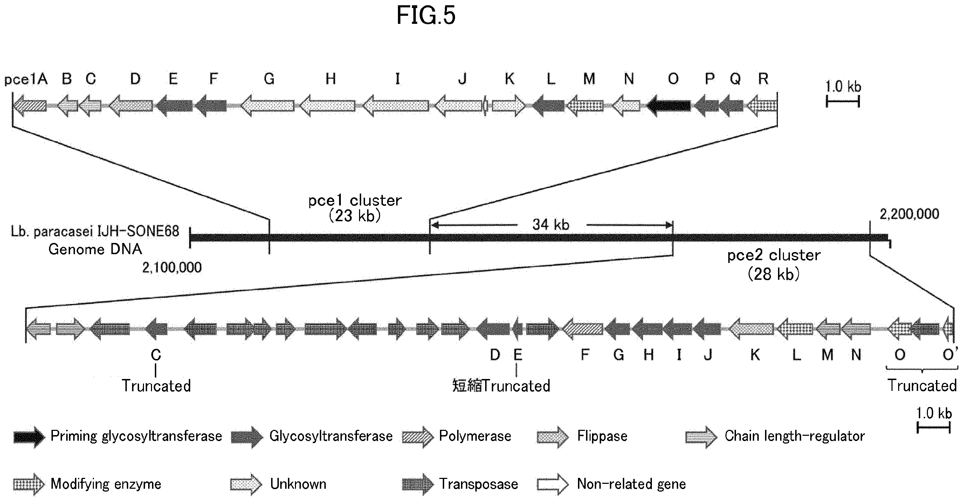

Fig. 5 is a structural diagram of exopolysaccharide biosynthetic gene clusters, which are named pce1 cluster and pce2 cluster, of genomic DNA of *Lactobacillus paracasei* strain IJH-SONE68.

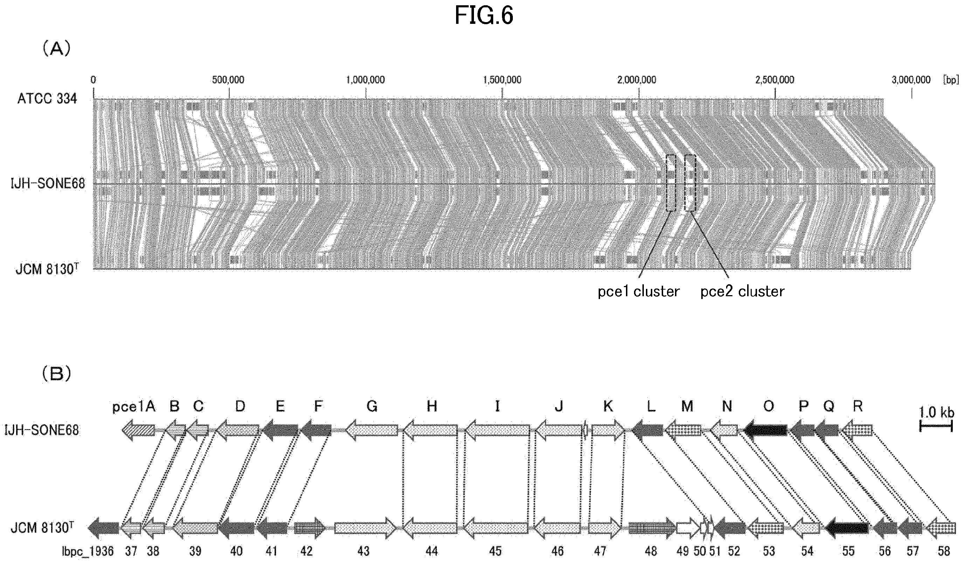

Fig. 6 illustrates at (A) genome rearrangement maps among three lactic acid bacteria of *Lactobacillus paracasei* strain IJH-SONE68, strain ATCC334, and strain JCM8130T. Fig. 6 illustrates at (B) the correspondences between pce1 gene cluster of *Lactobacillus paracasei* strain IJH-SONE68 and a gene cluster of strain JCM8130T.

Embodiments for Carrying out Invention

The exopolysaccharide of the present invention and use thereof are described below in detail.

1. The lactic acid bacterium of the present invention

The lactic acid bacterium producing the exopolysaccharide of the present invention is derived from a fig and belongs to *Lactobacillus paracasei*. The lactic acid bacterium is isolated from leaves, stems, fruits and the like of a fig. The lactic acid bacterium producing the exopolysaccharide of the present invention belongs to *Lactobacillus paracasei,* but not limited only to specific bacterial strains.

Specifically, according to the present invention, *Lactobacillus paracasei* strain IJH-SONE68 was isolated and identified from leaves of a fig, as a lactic acid bacterium that produces, as an exopolysaccharide, a neutral polysaccharide having a structure in which N-acetylglucosamines are linked with each other via $\alpha$-1,6 bond. This strain was nationally deposited under the accession number of NITE P-02242 at Patent Microorganisms Depositary, National Institute of Technology and Evaluation (#122, 2-5-8 Kazusakamatari, Kisarazu-shi, Chiba 292-0818, Japan) on April 19, 2016. The deposition was then transferred to an international deposit under the Budapest Treaty and given the international deposit accession number of NITE BP-02242 on May 26, 2017.

As illustrated in the photograph of Fig. 1, *Lactobacillus paracasei* strain IJH-SONE68 isolated and identified from leaves of a fig is a catalase-negative, gram-positive bacillus, and has mycological properties of forming a white colony and the characteristic of conditional heterolactic fermentation. Furthermore, the strain has an ability to produce a polysaccharide, in particular, a neutral polysaccharide having a structure in which N-acetylglucosamines are linked with each other via $\alpha$-1,6 bond.

This neutral polysaccharide is obtained by separating and purifying polysaccharides obtained from the culture of *Lactobacillus paracasei* strain IJH-SONE68, according to an anion exchange chromatography, as described in Example 4 provided hereinbelow. The NMR profiles of proton-NMR and carbon-NMR as illustrated in Fig. 3 have revealed that this neutral polysaccharide has a structure in which N-acetylglucosamines are linked with each other via $\alpha$-1,6 bond. In addition, *Lactobacillus paracasei* strain IJH-SONE68 extracellularly secretes an acidic polysaccharide composed mainly of glucoses and mannoses. More specifically, the acidic polysaccharide is composed of glucoses, mannoses, galactoses and rhamnoses, and the composition ratio is about 10:170:2:1.

The neutral and acidic polysaccharides have an activity of inhibiting hyaluronidase that is an enzyme hydrolyzing hyaluronic acid, as described in Example 4 provided hereinbelow.

In addition, *Lactobacillus paracasei* strain IJH-SONE68 has an ability of assimilating sugars, as shown in Table 1 of Example 3 provided hereinafter. In particular, as compared with other *Lactobacillus paracasei*, *Lactobacillus paracasei* strain IJH-SONE68 has a sugar-assimilating ability characterized in that it cannot assimilate amygdalin that may generate hydrocyanic acid when decomposed, or arbutin that is reported to inhibit melanin production thereby to exert a whitening effect.

From analysis of the whole genome sequence of *Lactobacillus paracasei* strain IJH-SONE68, it has been predicted that the genomic DNA of *Lactobacillus paracasei* strain IJH-SONE68 consists of 3,084,917 bp with a GC content of 46.37%, and has 2,963 structural genes. Furthermore, it has been shown that *Lactobacillus paracasei* strain IJH-SONE68 has two plasmids, one of which has a size of at least 51 kb, and the other has a size of 45,267 bp. As compared with other lactic acid bacteria, *Lactobacillus paracasei* strain IJH-SONE68 has a larger genome size and the larger number of structural genes.

In addition, two exopolysaccharide biosynthesis gene clusters have been found in the genomic DNA sequence of *Lactobacillus paracasei* strain IJH-SONE68, one of the two clusters is 23 kb cluster which has been named pce1 cluster, and other cluster is 28 kb cluster which has been named pce2 cluster. It has been then found that a protein deduced from a gene, which is one of glycosyltransferase genes in the pce2 cluster and which has been named pce2J, has a motif or domain similar to pfam02485 motif or domain observed in β-1,6-N-acetylglucosaminyltransferase that has already been known (Genes Dev., 1993 Mar; 7(3): 468-478, and J. Biol. Chem., 1999 Jan 29; 274(5): 3215-3221). Hence, it has been suggested that this structural gene in pce2 cluster is involved in a biosynthesis of the neutral polysaccharide.

In the present invention, the lactic acid bacterium of the present invention also includes a lactic acid bacterium equivalent to *Lactobacillus paracasei* strain IJH-SONE68. Here, the equivalent lactic acid bacterium indicates a lactic acid bacterium that belongs to *Lactobacillus paracasei* and has an ability of producing a neutral polysaccharide having a structure in which N-acetylglucosamines are linked with each other via α-1,6 bond. In addition, the equivalent lactic acid bacterium indicates a bacterial strain which belongs to *Lactobacillus paracasei,* 16S rDNA gene of which has a base sequence having 98% or more, preferably 99% or more, more preferably 100% identity with the base sequence of SEQ ID NO: 1 of 16S rDNA gene of *Lactobacillus paracasei* strain IJH-SONE68, and which preferably has the same microbial properties and/or the same sugar-assimilating ability as those of *Lactobacillus paracasei* strain IJH-SONE68. In addition, the equivalent lactic acid bacterium indicates a bacterial strain that produces an exopolysaccharide having a hyaluronidase inhibitory activity, like the exopolysaccharide produced by *Lactobacillus paracasei* strain IJH-SONE68.

These equivalent lactic acid bacteria are obtained, for example, by performing usual mutation treatment technique, such as mutation and genetic recombination, on *Lactobacillus paracasei* strain IJH-SONE68 and, in addition, may be bacterial strains that has been bred by selecting natural mutation strains of *Lactobacillus paracasei* strain IJH-SONE68, and the like.

2. Obtainment and proliferation of the lactic acid bacterium of the present invention

The lactic acid bacterium of the present invention is obtained from a fig in the same manner as *Lactobacillus paracasei* strain IJH-SONE68 as described in Example 4 provided hereinafter.

The lactic acid bacterium of the present invention can be easily proliferated by culturing those obtained bacteria. The culture method is not limited to a specific one as long as it is capable of proliferating a lactic acid bacterium, and a method commonly used for culturing a lactic acid bacterium may be used as it is, or a method that is appropriately modified if necessary may be used. For example, the culture temperature may be usually 25 to 50°C, preferably 35 to 42°C. The cultivation may be performed under either aerobic or anaerobic condition, particularly preferably under anaerobic condition. For example, the cultivation may be performed while ventilating anaerobic gas such as carbon dioxide gas or nitrogen gas at an appropriate concentration. In addition, the cultivation may be also performed under microaerobic condition such as liquid static culture.

The medium for culturing a lactic acid bacterium is not particularly limited, but a medium usually used for culturing a lactic acid bacterium may be appropriately modified if necessary, and used. That is, for example, sugars such as galactose, glucose, fructose, mannose, sorbose, mannitol, salicin, cellobiose, maltose, sucrose, trehalose, starch hydrolyzate and molasses may be used as carbon sources depending on their assimilability. For example, ammonium salts and ammonium nitrates such as ammonia, ammonium sulfate, ammonium chloride and ammonium nitrate may be used as nitrogen sources. For example, sodium chloride, potassium chloride, potassium phosphate, magnesium sulfate, calcium chloride, calcium nitrate, manganese chloride, ferrous sulfate and the like may be used as inorganic salts. In addition, organic components such as peptone, sake cake, whey, soybean powder, defatted soybean meal, meat extract and yeast extract may be used. In addition, for example, MRS medium or a modified medium thereof may be suitably used as an already prepared medium.

3. Isolation and purification of the exopolysaccharide

The exopolysaccharide of the present invention is obtained by culturing the aforementioned lactic acid bacterium derived from fig and belonging to *Lactobacillus paracasei* in the aforementioned method and, for example, isolating and purifying polysaccharides obtained from the culture, by an ion exchange chromatography.

More specifically, the exopolysaccharide of the present invention is obtained, for example, by the steps of (1) removing bacterial cell bodies from the liquid culture by centrifugation; (2) recovering polysaccharides and proteins as precipitates from the culture obtained in the step (1), by precipitation due to ethanol or acetone; (3) removing the proteins from the recovered precipitates to recover exopolysaccharides; and (4) isolating and purifying the recovered exopolysaccharides by an anion-exchange chromatography.

In the steps, the aforementioned step (3) of removing the proteins from the recovered precipitates to recover exopolysaccharides includes, for example, methods of 1) precipitating the proteins with an aqueous trichloroacetic acid solution and removing the precipitated proteins by centrifugation; 2) thermally denaturing the proteins with heat treatment and removing the denatured proteins by centrifugation; and 3) performing the method 2), followed by proteolysis with proteinase and/or decomposition treatment of nucleic acids with DNase. In addition, when isolating and purifying the exopolysaccharide by an anion exchange chromatography, neutral and acidic polysaccharides are each separated and purified by eluting the exopolysaccharide with, for example, a concentration gradient eluate of NaCl.

In addition, these steps (1) to (4) may be performed plural times, or part of them may be omitted. Furthermore, dialysis, gel filtration, ultrafiltration and the like may also be combined if necessary. The thus obtained exopolysaccharide may be subjected to additional operations such as lyophilization if necessary.

4. Use of the exopolysaccharide of the present invention

The exopolysaccharide of the present invention exhibits an activity of inhibiting hyaluronidase that is an enzyme hydrolyzing hyaluronic acid. The exopolysaccharide of the present invention can be widely used as an active ingredient in various compositions including a food and drink composition, a pharmaceutical composition, a feed composition and a cosmetic composition. For example, it can be used as an active ingredient of a food and drink composition, a pharmaceutical composition, a feed composition or a cosmetic composition which are each used for hyaluronidase inhibition, antiallergy and the like.

When the exopolysaccharide of the present invention is used in such use, a culture obtained by culturing the lactic acid bacterium producing the exopolysaccharide can be used as the exopolysaccharide. In such case, the obtained culture may be diluted or concentrated and used, or bacterial cells recovered from the culture may be used. As long as the effect of the present invention is not impaired, various additional operations such as heating and freeze-drying may also be performed after the cultivation. The additional operations are preferably those enabling a high survival rate of the lactic acid bacterium after performed. The lactic acid bacterium may be viable or dead, and may include both viable and dead. The dead bacterium may be crushed.

The pharmaceutical composition of the present invention is not particularly limited as long as it contains the exopolysaccharide of the present invention. The pharmaceutical composition of the present invention is usually used by blending the exopolysaccharide of the present invention, the lactic acid bacterium producing the same, or the like with a physiologically acceptable liquid or solid pharmaceutical carrier, followed by formulation.

The dosage form of the pharmaceutical composition of the present invention is not particularly limited, but specific examples of the dosage form include tablets, pills, powders, solutions, suspensions, emulsions, granules, capsules, syrups, suppositories, injections, ointments, patches, eye drops, and nose drops. In the formulation, additives, such as excipients, binders, disintegrants, lubricants, stabilizers, flavoring agents, diluents, surfactants, and solvents for injections, commonly used as pharmaceutical carriers may be used.

The content of the exopolysaccharide, the lactic acid bacterium producing the same, or the like in the pharmaceutical composition of the present invention may be appropriately determined depending on the dosage form, the dosage regimen, the age and sex of a subject, the kind of disease, the degree of disease, other conditions and the like, but is usually preferably, for example, 0.001% or more by weight and 0.01% or more by weight in terms of the weight of the exopolysaccharide.

As long as the effect of the present invention is not impaired, the exopolysaccharide of the present invention may be appropriately used in combination with other active ingredient, for example, an immunostimulant.

The administration timing of the pharmaceutical composition of the present invention is not particularly limited, but may be appropriately selected according to a subject to be applied. The pharmaceutical composition of the present invention may also be administered prophylactically or used for a health maintenance. The administration mode may be preferably appropriately determined according to the dosage form, age, sex and other conditions of the administered subject, the degree of symptoms of the administered subject, and the like. In any case, the pharmaceutical composition of the present invention may be administered once per day or administered dividedly into a plurality of times, or administered once every several days or weeks.

The pharmaceutical composition of the present invention may be used, for example, to lower the allergy of a subject to be administered.

The food and drink of the food and drink composition containing the exopolysaccharide of the present invention, the culture of the lactic acid bacterium producing the same or the like are not particularly limited as long as they contain the exopolysaccharide of the present invention, but examples of the food and drink include beverages such as soft drinks, carbonated drinks, nutritional drinks, fruit juice beverages, and lactic acid bacteria beverages, concentrated stock solutions of these beverages, powders for the preparation of these beverages, and the like; ice cream, sherbet and ice confectionery such as shaved ice; confectioneries such as candy, gummy, cereal, chewing gum, candy, gum, chocolate, tablet candy, snack, biscuit, jelly, jam, cream, and baked confectionery; dairy products such as processed milk, milk drink, fermented milk, drink yogurt, and butter; bread; enteral nutritious food, liquid food, childcare milk, sports drink;

food such as puree; and other functional foods. In addition, the food and drink may be supplements, and the supplements may be in the form of, for example, granules, powders, or tablets. In the case of supplements, the lactic acid bacterium may be ingested without being affected by other foods with respect to the amount of meal and calorie intake per day.

The food and drink as described above may be prepared by adding the exopolysaccharide, the culture of the lactic acid bacterium producing the same, or the like to raw materials of food and drink, or prepared in the same manner as usual food and drink. The addition of the exopolysaccharide or the culture of the lactic acid bacterium producing the same may be performed at any stage of the process of preparing the food and drink. The food and drink may be prepared after a fermentation process of the added exopolysaccharide or culture. Examples of such food and drink include fermented foods such as lactic acid bacterium beverages and fermented milks. As raw materials for the food and drink, raw materials used for usual foods and drinks may be used. The prepared food and drink may be ingested orally.

The food and drink of the present invention include raw materials for preparing the food and drink, and food additives or the like added to the food and drink during the preparation processes or after the preparation processes. For example, the exopolysaccharide of the present invention, the culture of the lactic acid bacterium producing the same, and the like may be used as a starter for preparing fermented milks. In addition, the exopolysaccharide of the present invention, the culture of the lactic acid bacterium producing the same, or the like may be added to the fermented milks after prepared.

The content of the exopolysaccharide of the present invention, the culture of the lactic acid bacterium producing the same, or the like in the food and drink composition may be appropriately determined depending on the embodiment of the food and drink, but is usually 0.001 % or more by weight, preferably 0.01% or more by weight, in the food and drink in terms of the weight of the exopolysaccharide.

The food and drink composition containing the exopolysaccharide of the present invention can be used in various uses utilizing the antiallergy effect or anti-alcoholic injury effect.

The food and drink containing the exopolysaccharide of the present invention, the culture of the lactic acid bacterium producing the same, or the like may be manufactured and sold as a food and drink showing its use. Such a food and drink may be showed by "for allergy improvement", and the like. Other showing may also be used, needless to say, as long as it indicates the secondary effect caused by such improvement effect. The term "show" as used herein means all actions for informing a consumer of the aforementioned use, and any actions fall under the showing, regardless of the purpose and content of the showing, a subject and medium to be showed, and the like, as long as they recall or infer the aforementioned use. However, the showing is preferably made by an expression such that a consumer can directly recognize the aforementioned use.

Specifically, it may be exemplified that the aforementioned use is showed on a commodity or a package thereof regarding the food and drink of the present invention. In particular, the use is preferably showed on advertisement materials at sales sites and other documents, such as packages, containers, catalogs, pamphlets and POPs. Examples of the showed commodities include health foods, functional foods, enteral nutrition foods, special use foods, nutritional functional foods, quasi drugs, and special health foods.

Examples of the feed of a feed composition containing the exopolysaccharide of the present invention, the culture of the lactic acid bacterium producing the same, or the like include pet food, livestock feed and fish feed. Such a feed may be prepared by mixing common feed, for example, cereals, cakes, brans, fish meals, bone meals, oils and fats, skim milk powders, wheys, bitterns, mineral feeds, yeasts, and the like with the exopolysaccharide of the present invention, the culture of the lactic acid bacterium producing the same, or the like. In addition, for example, likewise the case of silage, a feed may be prepared through a fermentation process with the lactic acid bacterium added thereto. The prepared feed may be orally administered to general mammals, livestock, farmed fishes, pet animals and the like. In the case of farmed fishes, it may be adopted to spread fermented products, to which the lactic acid bacterium of the present invention, to the farmed place of fishes.

The content of the exopolysaccharide of the present invention, the culture of the lactic acid bacterium producing the same, or the like in the feed composition may be appropriately determined depending on the embodiment of the feed or the administered subject, but is usually 0.001% or more by weight, preferably 0.01% or more by weight, in terms of the weight of the exopolysaccharide.

The feed composition containing the exopolysaccharide of the present invention, the culture of the lactic acid bacterium producing the same, or the like can be used in various uses utilizing, for example, the antiallergy effect.

Examples of the cosmetic product of the cosmetic composition containing the exopolysaccharide of the present invention, the culture of the lactic acid bacterium producing the same, or the like include washing agents such as soaps, body shampoos, cleansing creams and facial cleansers; creams such as lotions, vanishing creams, cold creams, emollient creams and massage creams; milky lotions and serums.

The content of the exopolysaccharide of the present invention, the culture of the lactic acid bacterium producing the same, or the like used in the cosmetic composition may be appropriately determined depending on the embodiment of the cosmetic or the applied site, but is, for example, usually 0.001% or more by weight, preferably 0.01% or more by weight, in terms of the weight of the exopolysaccharide.

The cosmetic composition containing the exopolysaccharide of the present invention, the culture of the lactic acid bacterium producing the same, or the like can be used in various uses utilizing, for example, the antiallergy effect.

Examples

Hereinafter, the present invention will be described in more detail with reference to examples, but the present invention is not limited by these examples.

Example 1

Isolation and identification of lactic acid bacterium

1. Isolation of lactic acid bacterium sample

The leaves, stems and fruits of a fig (variety "TOYOMITSU HIME") were chosen and cut into pieces of 2 to 3 mm using sterilized tweezers and scissors. Every five to six pieces were then placed in a sterilized test tube containing MRS liquid medium, and statically cultured at 28°C and 37°C until the MRS medium as a standard medium for a lactic acid bacterium became turbid (proliferated). By the way, it took 2 to 4 days for the proliferation of the lactic acid bacterium candidate strains to be visible.

A part of each culture liquid of the lactic acid bacterium candidate strains was subjected to a line drawing paint on MRS agar medium using a disposable loop, followed by stationary culture. Among colonies formed on the agar medium, all of differently colored, lustrous and shaped colonies were picked up and subjected to a line drawing paint on a fresh MRS agar medium, and the colonies were purified.

$H_2O_2$ test was performed for each purified colony to verify the presence or absence of the production of a catalase enzyme. This is a test method for observing the presence or absence of oxygen generated when catalase is present, which is observed when cell bodies are exposed to 10% $H_2O_2$ solution. By the way, a lactic acid bacterium produces no catalase.

As a result of attempting the search and isolation from a fig, one lactic acid bacterium candidate strain showing catalase-negative was obtained from the leaves of a fig as the isolation source.

2. Identification of the isolated strain

The aforementioned lactic acid bacterium candidate strain was again cultured in MRS liquid medium, and the bacterial cell bodies were obtained by centrifugation. After the cell bodies were treated with cell wall lytic enzyme, a genomic DNA was extracted using DNAzol reagent.

According to the method as described in Lane, DJ (1991), "16S/23S rRNA sequencing", Nucleic Acid Techniques in Bacterial Systematics, pp. 115-175, edited by E. Stackebrandt & M. Goodfellow. Chichester: Wiley, a genomic DNA PCR was performed using a genomic DNA as a template and using 27f primer (5'-AGAGTTTGATCCTGGCTCAG-3') (SEQ ID NO: 1 in Sequence Listing) and 1525r primer (5'-AAAGGAGGTGATCCAGCC-3') (SEQ ID NO: 2 in Sequence Listing), thereby to amplify 16S rDNA part. Then, an objective fragment was recovered from agarose gel according to NucleoSpin Gel and PCR Clean-up kit (manufactured by Mahalay Nagel). A sequencing reaction by a dye terminator method for sequencing a base sequence was performed with Big Dye Terminator Cycle Sequencing FS Ready Reaction Kit ver. 3.1 (manufactured by ThermoFisher Scientific), and analysis was made with ABI PRISM 3130 xl Genetic Analyzer (manufactured by ThermoFisher Scientific). The base sequence of the analyzed 16S rDNA had the base sequence of SEQ ID NO: 3 in Sequence Listing. The base sequence was subjected to a homology search by BLAST program and compared with the database of DNA data bank (DDBJ/EMBL/GenBank) to make a taxonomic identification on the isolated strain.

The lactic acid bacterium candidate strain isolated from leaves of a fig was named strain IJH-SONE68 and identified as *Lactobacillus paracasei* because it was 100% identical to a base sequence which was in the strain of *Lactobacillus paracasei* R094 already registered in DNA data bank (DDBJ/EMBL/GenBank) and which had NR-025880 as the accession number of the base sequence.

This strain was nationally deposited under the accession number of NITE P-02242 at Patent Microorganisms Depositary, National Institute of Technology and Evaluation (#122, 2-5-8 Kazusakamatari, Kisarazu-shi, Chiba 292-0818, Japan) on April 19, 2016. The deposition was then transferred to an international deposit under the Budapest Treaty and given the international deposit accession number of NITE BP-02242 on May 26, 2017.

3. Sequence analysis of genomic DNA

The genomic DNA sequence from the strain IJH-SONE68 was sequenced by PacBio RS II (Pacific Biosciences, Menlo Park, CA, USA) on a single molecule real-time (SMRT) cell using P6 polymerase and C4 chemistry (P6C4). The purified genomic DNA sample was sheared into fragments using g-TUBE Kit (Covaris, Woburn, MA, USA). The sheared fragments were then purified using AMPure PB Kit (Pacific Biosciences). DNA library was constructed using PacBio DNA Template Prep Kit 1.0 (Pacific Biosciences) and PacBio DNA/Polymerase Binding Kit P6 (Pacific Biosciences). The short fragments were removed using Blue Pippin (Sage Science, Beverly, MA, USA), and the purified DNA library was then sequenced on PacBio SMRT Platform. De novo assembly was performed according to the protocol of Hierarchical Genome Assembly Process (HGAP) (Nat. Methods, 10, 563-56933), and the obtained whole genome contig was annotated by Microbial Genome Annotation Pipeline (MiGAP) (The 20th International Conference on Genome Informatics (GIW2009) Poster and Software Demonstrations (Yokohama), S001-1-2).

4. Results of the sequence analysis of the genomic DNA

The whole genome sequence of the strain IJH-SONE68 was sequenced and, as a result, the genomic DNA consisted of 3,084,917 bp with GC content of 46.37%, and the number of structural genes was predicted to be 2,963 according to MiGAP. Furthermore, it was shown from the results that the strain IJH-SONE68 harbored two plasmids, one of which had a size of at least 51 kb, and the other had a size of 45,267 bp. The strain IJH-SONE68 had a larger genome size and the larger number of structural genes, as compared with other lactic acid bacteria.

Example 2

Mycological properties of the isolated and identified lactic acid bacterium

The aforementioned isolated and identified lactic acid bacterium strain IJH-SONE68 was a catalase-negative, gram-positive bacillus and had a white colony forming property, as illustrated in the photograph of Fig. 1, and further had the characteristic of conditional hetero-lactic acid fermentation and the ability of producing polysaccharides.

Example 3

Saccharide assimilation ability of the isolated and identified lactic acid bacterium

1. Test method of assimilation ability

The strain IJH-SONE68 was investigated for the assimilation ability of 49 kinds of saccharides according to the following test method.
The strain IJH-SONE68 was statically cultured in MRS liquid medium until the proliferation stationary phase. The bacterial cell bodies obtained by centrifugation were washed with an appropriate amount of a suspension medium (manufactured by BioMerieux), and finally suspended in 2 mL of a suspension medium. A portion of the resultant suspension was added to 5 mL of a suspension medium to determine an amount (n) for McFarland turbidity to become 2. Subsequently, 2n of a bacterial solution was added to API 50 CHL medium (manufactured by BioMerieux), and this solution was dispended to each well of API 50 CHL Kit (manufactured by BioMerieux, 49 kinds of saccharide were coated on the bottom of each well). Finally, mineral oil was overlaid and set in a tray containing a sterilized water. After culturing at 37°C for 48 hours, the presence or absence of the assimilation ability was assessed by observing the change in color tone in each well.

2. Test results of the assimilation ability

Table 1 shows the results of investigating the assimilation ability of the strain IJH-SONE68 against 49 kinds of saccharides. Table 1 also shows the results of investigating the assimilation ability of other *Lactobacillus paracasei* strains described in patent-laid open publications using similar kits.

[Table 1]

| | | | | | | | |
|---|---|---|---|---|---|---|---|
| Assimilation abilities of the strain IJH-SONE68 against saccharides | | | | | | | |
| Substrates | IJH-SONE 68 | JP2016-123382 | JP2007-189973 | JP2007-189973 | JP2016-113378 | JP2016-37451 | JP2011-142907 |
| | NITE BP-02242 | NITE P-01960 | NLB162 NITEP-159 | NLB163 NITEP-160 | MCC1849 NITE BP-01633 | HL190 NITE P-01810 | LT12 NRRL-B50327 |
| control | - | - | - | - | - | | - |
| glycerol | - | - | - | - | - | - | - |
| erythritol | - | - | - | - | - | - | - |
| D-arabinose | - | - | - | - | - | - | - |
| L-arabinose | - | - | - | - | - | - | - |
| D-ribose | + | + | + | + | + | + | + |
| D-xylose | - | - | - | - | - | - | - |
| L-xylose | - | - | - | - | - | - | - |
| D-adonitol | + | - | - | - | - | - | - |
| methyl-βD-xylopyranoside | - | - | - | - | - | - | - |
| D-galactose | + | + | + | + | + | + | + |
| D-glucose | + | + | + | + | + | + | + |
| D-fructose | + | + | + | + | + | + | + |
| D-mannose | + | + | + | + | + | + | + |
| L-sorbose | + | - | + | + | - | - | + |
| L-rhamnose | - | + | - | - | - | - | - |
| dulcitol | - | - | - | - | - | - | + |
| inositol | - | - | - | - | - | - | + |
| D-mannitol | + | + | + | + | + | - | + |
| D-sorbitol | - | + | + | - | + | - | + |

| Assimilation abilities of the strain IJH-SONE68 against saccharides | | | | | | |
|---|---|---|---|---|---|---|
| Substrates | IJH-SONE 68 | JP2016-123382 | JP2007-189973 | JP2007-189973 | JP2016-113378 | JP2016-37451 | JP2011-142907 |
|  | NITE BP-02242 | NITE P-01960 | NLB162 NITEP-159 | NLB163 NITEP-160 | MCC1849 NITE BP-01633 | HL190 NITE P-01810 | LT12 NRRL-B50327 |
| methyl-αD-mann opyranoside | - | - | + | - | - | - | - |
| methyl-αD-glucopyranoside | - | - | ± | + | ± | - | + |
| N-acetylglucosamine | + | + | + | + | ± | + | + |
| amygdalin | - | + | + | + | ± | + | + |
| arbutin | - | + | + | + | + | + | + |
| esculin + ferric citrate | + | + | ± | ± | + | + | |
| salicin | + | + | + | + | + | + | + |
| D-cellobiose | + | + | + | + | + | + | + |
| D-maltose | + | + | + | + | + | + | + |
| D-lactose | - | + | - | + | + | + | + |
| D-melibiose | - | - | - | - | - | - | - |
| D-sucrose | + | + | + | + | + | + | + |
| D-trehalose | + | + | + | + | + | + | + |
| inulin | - | + | - | - | + | + | + |
| D-melezitose | + | + | + | + | + | - | + |
| D-raffinose | - | - | - | - | - | - | - |
| starch | - | - | - | - | - | - | - |
| glycogen | - | - | - | - | - | - | - |

(continued)

| Assimilation abilities of the strain IJH-SONE68 against saccharides | | | | | | | |
|---|---|---|---|---|---|---|---|
| Substrates | IJH-SONE 68 | JP2016-123382 | JP2007-189973 | JP2007-189973 | JP2016-113378 | JP2016-37451 | JP2011-142907 |
| | NITE BP-02242 | NITE P-01960 | NLB162 NITEP-159 | NLB163 NITEP-160 | MCC1849 NITE BP-01633 | HL190 NITE P-01810 | LT12 NRRL-B50327 |
| xylitol | - | - | - | - | - | - | - |
| gentibiose | + | + | + | + | ± | + | + |
| D-turranose | - | + | + | + | + | + | + |
| D-lyxose | - | - | ± | - | - | - | + |
| D-tagatose | + | + | + | + | + | + | + |
| D-fucose | - | - | - | - | - | - | - |
| L-fucose | - | - | - | - | - | - | - |
| D-arabitol | - | - | - | - | - | - | - |
| L-arabitol | - | - | - | - | - | - | - |
| gluconic acid | + | + | ± | ± | ± | + | + |
| 2-ketogluconic acid | - | - | - | - | - | | - |
| 5-ketogluconic acid | - | - | - | - | - | | - |

In Table 1, + indicates the possession of assimilation ability, and - indicates no possession of assimilation ability.

Saccharide assimilation kit: Apl50CHL (manufactured by bioMerieux) was used in JP2016-123382 and JP2011-142907, AIP50CH (manufactured by Simex·BioMerieux) was used in JP2016-113378, and there are no descriptions for kits used in JP2007-189973 and JP2016-37451.

NITE P-01960, NITE PB-01633 and NRRL-B50327 are *Lactobacillus paracasei,* and NITE P-159, NITE P-160 and NITE P-01810 are *Lactobacillus paracasei* ssp. *paracasei.*

As can be seen from the results of Table 1, when compared with other *Lactobacillus paracasei* strains, the strain IJH-SONE68 cannot assimilate amygdalin that may generate hydrocyanic acid when decomposed, or arbutin that is reported to inhibit melanin production thereby to exert a whitening effect, and thus decomposes neither amygdalin nor arbutin. Hence, the strain IJH-SONE68 can be said to be excellent in the safety, and also excellent in the whitening effect when used as an additive for cosmetics. In addition, while other *Lactobacillus paracasei* strains cannot assimilate D-adonitol, but can assimilate D-chulanose, the strain IJH-SONE68 has the characteristics of being able to assimilate D-adonitol, but unable to assimilate D-chulanose.

Example 4

1. Isolation and purification of exopolysaccharides produced by the strain

IJH-SONE68

Exopolysaccharides produced by the strain IJH-SONE68 were isolated and purified according to the following method.
The strain IJH-SONE68 was statically cultured in MRS liquid medium until the proliferation stationary phase. 5 mL of the resultant culture solution was used as a seed culture solution, and inoculated on 5 L of a semisynthetic medium for producing exopolysaccharides (the composition thereof will be described below), followed by static culture at 37°C for 120 hours. After the resultant culture solution was cooled to 4°C, proteins contained in the culture supernatant were denatured, and 202.5 mL of a 100% trichloroacetic acid aqueous solution was added thereto, mixed and allowed to stand for 30 minutes to remove them as precipitates in a later step. After the precipitates were removed by centrifugation, an equal amount of acetone was added to the collected supernatant and mixed, and the resultant mixture was allowed to stand at 4°C overnight to precipitate polysaccharides produced by the strain IJH-SONE68. The precipitates were collected by centrifugation, and the resultant precipitates were then washed with 250 mL of 70% ethanol. After the precipitates were air-dried, 75 mL of 50 mM Tris-HCl buffer (pH 8.0) was added to the resultant precipitates, and mixed for 1 hour to dissolve the precipitates. After insoluble impurities were removed by centrifugation to recover a supernatant, 750 μL of 1 mg/mL DNase solution (Worthington, Inc.) and 750 μL of 1 mg/mL RNase solution (Nacalai Tesque, Inc.) were each added to the recovered supernatant, followed by being allowed to react at 37°C for 8 hours. Subsequently, 750 μL of 2 mg/mL proteinase K solution (manufactured by Wako Pure Chemical Industries, Ltd.) was added, and the resultant mixture was reacted at 37°C for 16 hours. The resultant solution after the reaction was cooled to 4°C, the added enzymes were each denatured, and 8.75 mL of a 100% trichloroacetic acid aqueous solution was then added thereto, mixed and allowed to stand for 1 hour to remove the enzymes as precipitates in the next centrifugation. The resultant precipitates were removed by centrifugation to obtain a supernatant, 262.5 mL of 100% ethanol was added to the obtained supernatant, the resultant mixture was thoroughly mixed, and the polysaccharides produced by the strain IJH-SONE68 strain were then recovered as precipitates by centrifugation. After the precipitates were washed with 50 mL of 70% ethanol, the precipitates were air-dried, an appropriate amount (about 25 mL) of a purified water was added thereto, and the resultant mixture was allowed to stand overnight at 4°C to dissolve the polysaccharides. For the polysaccharide sample after the dissolution, small molecules such as monosaccharides in the recovered sample were removed using an ultrafiltration unit (Merck Ltd.) of 10,000 MWCO while replacing the solvent with a purified water, and a purified polysaccharide sample was thus obtained.
The purified polysaccharide sample was applied to an open column (2.5 × 22 cm) packed with TOYOPEARL DEAE-650M resin (Tosoh Corporation) previously equilibrated with 50 mM Tris-HCl buffer (pH 8.0), and column work was performed to isolate and purify the sample to neutral polysaccharide fractions and acidic polysaccharide fractions. The same buffer was used as an elution solution, and a flow rate was fixed at 1 mL/min. In addition, eluates were collected in different test tubes at every 6 mL. First, from the beginning to 240 minutes, elution was made with the same buffer (Test Tube Nos. 1 to 40). Next, from 240 minutes to 600 minutes, a concentration gradient of 0 to 500 mM NaCl was prepared using the same buffer, and elution was continued with the gradient (Test Tube Nos. 41 to 100). The column isolation spectrum is illustrated in Fig. 2. After the presence of polysaccharides was confirmed by a phenol sulfuric acid method (described below) for all the samples eluted in the test tubes, the confirmed solutions in the test tubes were collected as neutral polysaccharide fractions and acidic polysaccharide fractions, respectively. For each fraction, an ultrafiltration unit of 10,000 MWCO was used to remove small molecules such as monosaccharides in the recovered sample while replacing the solvent with purified water.
As a result, neutral polysaccharide fractions and acidic polysaccharide fractions were separated and purified as exopolysaccharides produced by the strain IJH-SONE68.
A semisynthetic medium for producing polysaccharides was prepared by modifying a medium described in Kimmel SA, Roberts RF., "Development of a growth medium suitable for exopolysaccharide production by Lactobacillus delbrueckii ssp. Bulgaricus RR.", Int. J. Food Microbiol., 40, 87-92 (1998), as follows:

| Semisynthetic medium for producing polysaccharides | [g/L] |
|---|---|
| Glucose | 20 |
| Tween 80 | 1.0 |
| Ammonium citrate | 2.0 |
| Sodium acetate | 5.0 |
| $MgSO_4 \cdot 7H_2O$ | 0.1 |
| $MnSO_4 \cdot 5H_2O$ | 0.05 |
| $K_2HPO_4$ | 2.0 |
| Bacto casitone | 10.0 |
| Vitamin Soln. | 2 mL |
| Trace element Soln. | 1 mL |

| Vitamin Soln. | [g/L] |
|---|---|
| 4-Aminobenzoic acid | 0.05 |
| Biotin | 0.001 |
| Folic acid | 0.025 |
| Lipoic acid | 0.025 |
| Nicotinic acid | 0.1 |
| Pantothenic acid | 0.05 |
| Pyridoxamin-HCl | 0.25 |
| Vitamin $B_{12}$ | 0.05 |
| Pyridoxine | 0.025 |
| Riboflavin | 0.05 |
| Thiamine | 0.1 |

Trace element Soln. is described in Kets EPW, Galinski EA, de Bont JAM. Carnitine: "A novel compatible solute in Lactobacillus plantarum", Arch. Microbiol., 192, 243-248 (1994), and the composition is as follows:

| Trace element Soln. | [g/L] |
|---|---|
| 25% HCl | 10 mL |
| $FeCl_2 \cdot 4H_2O$ | 1.5 |
| $CoCl_2 \cdot 6H_2O$ | 0.19 |
| $MnCl_2 \cdot 4H_2O$ | 0.1 |
| $ZnCl_2$ | 0.07 |
| $H_3BO_3$ | 0.006 |
| $Na_2MoO_4 \cdot 2H_2O$ | 0.036 |
| $NiCl_2 \cdot 6H_2O$ | 0.024 |
| $CuCl_2 \cdot 2H_2O$ | 0.002 |

Phenol sulfuric acid method (DuBois M, Gilles KA, Hamilton JK, Rebers PA, Smith F., "Colorimetric method for determination of sugars and related substances", Anal. Chem., 28, 350-356 (1956))

30 μL of a subject sample was mixed with an equal amount of 5 w/v% phenol aqueous solution, and 150 μL of a concentrated sulfuric acid was added to the resultant mixture and mixed with each other to allow a reaction to start. Immediately after 10 minutes, the reaction solution was cooled by ice to stop the reaction. The concentration of saccharides was obtained by measuring the absorbance of the reaction solution at 490 nm. The concentration was determined using a calibration curve prepared by performing the same experiment using glucose as a standard.

2. Structural analysis of neutral exopolysaccharide

The neutral exopolysaccharide purified by the aforementioned anion exchange column chromatography (TOYOPEARL DEAE-650 M resin (Tosoh Corporation)) was subjected to proton-NMR and carbon-NMR, and the obtained NMR profiles are each illustrated in Fig. 3. The structural analysis results of the neutral exopolysaccharide from these NMR profiles are illustrated in Fig. 4.

From the structural analysis results, it was revealed that the neutral exopolysaccharide produced by the strain IJH-SONE68 has a structure in which N-acetylglucosamines are linked with each other via $\alpha$-1,6 bond.

3. Saccharide composition analysis of acidic exopolysaccharide

The saccharide composition analysis of the aforementioned acidic exopolysaccharide purified by the anion exchange column chromatography was performed by measuring the composition by a high performance liquid chromatography (HPLC) method.

A 7-fold diluted sample solution was prepared by mixing 10 $\mu$L of the purified acidic exopolysaccharide (7.3 mg/mL) and 60 $\mu$L of water, and placed in a test tube. 20 $\mu$L of the diluted sample solution was collected from the test tube, dried under reduced pressure, and 100 $\mu$L of 2 mol/L trifluoroacetic acid was added thereto to dissolve the dried sample. The resultant solution was substituted with nitrogen, sealed under a reduced pressure, hydrolyzed at 100°C for 6 hours, and then dried under a reduced pressure. To the obtained residue, 200 $\mu$L of water was added, dissolved, and filtrated with 0.22 $\mu$m filter, to obtain a sample solution for measurement. The sample solution for measurement was 10-fold diluted with water to obtain a sample solution for dilution measurement. 50 $\mu$L of each of these sample solutions was analyzed. HPLC system: LC-20A system (Shimadzu Corporation) and spectrofluorophotometer M-10AxL (Shimadzu Corporation) were used as analytical instruments. The analysis conditions were as follows:

Column: TSK-gel Sugar AXG 4.6 mml. D. $\times$ 15 cm (Tosoh Corporation)
Column temperature: 70°C
Mobile phase: 0.5 mol/L potassium borate buffer, pH 8.7
Mobile phase flow rate: 0.4 mL/min
Post column labeling: reaction reagent: 1 w/v% arginine·3 w/v% boric acid
Reaction reagent flow rate: 0.5 mL/min
Reaction temperature: 150°C
Detection wavelength: Ex. 320 nm, Em. 430 nm

Standard solutions of acidic saccharides, chromatograms of samples, calibration curves of acidic saccharides and calibration curve data were obtained. The concentrations of constituent saccharides of the acidic exopolysaccharide in the sample were determined from calibration curves. The obtained results are shown in Table 2.

[Table 2]

| Constituent saccharides of acidic exopolysaccharide | | |
|---|---|---|
| Acidic exopolysaccharide | | Concentration in sample (mg/mL) |
| Monosaccharides | Rhamnose | 0.0204 |
| | Ribose | n.d. |
| | Mannose | 3.43 |
| | Arabinose | n.d. |
| | Galactose | 0.0384 |
| | Xylose | n.d. |
| | Glucose | 0.219 |
| In the Table, n.d. indicates no detection. | | |

4. Analysis of exopolysaccharide-biosynthesizing gene cluster of the strain IJH-SONE68

Based on the annotation of the genome sequence of the strain IJH-SONE68 as described in Example 1, two exopolysaccharide-biosynthesizing gene clusters were found in the genomic DNA (Fig 5). A gene cluster, which is one of the two clusters and which is 23 kb cluster, was named pce1 cluster, and the peel cluster included 18 open reading frames (ORFs) (pce1A to R) including unknown protein-encoding genes. The other gene cluster of 28 kb was designated pce2, and the pce2 cluster is composed of 12 complete ORFs and three truncated ORFs (pce2A to O). Furthermore, 12 transposase-related genes were found in the pce2 cluster. With respect to genes encoding proteins necessary for the biosynthesis of exopolysaccharides, wzb gene encoding protein-tyrosine phosphatase Wzb that acts as a chain-length factor (Yother J. Annu. Rev. Microbiol., 65, 563-581 (2011)) was not found in the pce1 cluster. On the other hand, a gene having a homology with priming glycosyltransferase that catalyzes the first step of saccharide polymerization (van Kranenburg R, Vos HR, van Swam II, Kleebezem M, de Vos WM., J. Bacteriol., 1999 Oct; 181(20): 6347-6353) was not present in the pce2 cluster.

The genome rearrangement map among three lactic acid bacteria of the strains IJH-SONE68, ATCC 334 (Makarova, K. et. al, Proc. Natl. Acad. Sci. U.S.A., 103 (42), 15611-15616 (2006)) and JCM 8130T (Toh, H. et. al, PLoS ONE 8, e75073 (2013)) was drawn up (Fig. 6). From the map, it was revealed that the pce2 cluster region is specific for the strain IJH-SONE68. On the other hand, a gene cluster homologous to pce2 cluster was not observed in the strain ATCC 334, but was present in the strain JCM 8130T.

Based on the aforementioned homology search, genes that were each homologous with wzb gene and priming glycosyltransferase gene were observed in the pce1 and pce2 clusters. Since other clusters or the like were not found in the genomic DNA of the strain IJH-SONE68, genes necessary for the biosynthesis of exopolysaccharides were considered to be complemented with the pce1 and pce2 clusters. In fact, the pce1 cluster and the pce2 cluster were only 34 kb apart from each other.

In the pce2 cluster, a protein deduced from one of glycosyltransferase genes, named pce2J, was found to have a motif or domain similar to pfam02485 motif or domain found in already known $\beta$-1,6-acetylglucosaminyltransferase (Genes Dev. 1993 Mar;7(3):468-478, and J. Biol. Chem., 1999 Jan 29;274(5):3215-3221), and this structural gene was suggested to be involved in the biosynthesis of the neutral exopolysaccharide. Indeed, the pce2 cluster was specific for the strain IJH-SONE68 as compared with the strains ATCC 334 and JCM 8130T, and a neutral polysaccharide having a new structure was considered to be biosynthesized from the pce2 cluster.

Example 5

Hyaluronidase activity inhibition of exopolysaccharides produced by the strain IJH-SONE68

A hyaluronidase activity inhibition was investigated on the polysaccharide sample containing the neutral polysaccharide fractions and the acidic polysaccharide fractions, the neutral polysaccharide fractions, and the acidic polysaccharide fractions, which were exopolysaccharides produced by the strain IJH-SONE68 and obtained in Example 4.

1. Test method

5 $\mu$L of a hyaluronidase enzyme solution (MP Biomedicals, 4 mg/mL, 100 mM sodium acetate buffer (pH 4.0)) was added to 10 $\mu$L of an aqueous solution containing polysaccharides at an optional concentration, which was prepared from the polysaccharide sample containing the neutral polysaccharide fractions and the acidic polysaccharide fractions, the neutral polysaccharide fractions, and the acidic polysaccharide fractions, which were exopolysaccharides produced by the strain IJH-SONE68 and obtained in Example 4. The resultant mixture was incubated at 37°C for 20 minutes. Thereafter, to the mixture, 10 $\mu$L of an enzyme-activating solution (0.5 mg/ml Compound 48/80 (manufactured by MP Biomedicals)), 3.75 mg $CaCl_2 \cdot 2H_2O$, and 100 mM sodium acetate buffer (pH 4.0)) were added, and incubated again at 37°C for 20 minutes. Subsequently, to the resultant mixture, 25 $\mu$L of a sodium hyaluronate solution (Wako Pure Chemical Industries, 0.8 mg/mL, 100 mM sodium acetate buffer (pH 4.0)) was added, and further reacted at 37°C for 40 minutes. After the reaction, the reaction was terminated by adding 10 $\mu$L of 0.4 M NaOH aqueous solution. Subsequently, to the reaction solution, 10 $\mu$L of 100 mM potassium borate buffer (pH 10.0) was added, and the mixture was heated at 100°C for 3 minutes, and immediately thereafter cooled with ice. 40 $\mu$L of the reaction solution was mixed with 200 $\mu$L of p-DMAB solution (described below), the mixture was reacted at 37°C for 20 minutes, and the absorbance at 585 nm was then measured. As a control, a reaction solution not containing a hyaluronidase enzyme solution was prepared and experimented in the same manner.

The inhibition rate of the enzyme activity for the polysaccharide sample was obtained from the following equation:

$$\text{Inhibition rate (\%)} = 100 - (S/C) \times 100$$

In this equation, C means the enzyme activity in the absence of the sample, and S means the enzyme activity in the presence of the sample. In addition, $IC_{50}$ value of the polysaccharide sample was obtained by obtaining a plurality of data on the changed content concentrations, plotting these data on X-axis as the concentration of the polysaccharide sample, and on Y-axis as the inhibition percentage, and obtaining the value from the following approximation equation:

[Equation 1]

$$Y = \alpha / (1 + \beta\, e^{-\gamma X})$$

In the equation, $\alpha$, $\beta$ and $\gamma$ are given constants.

p-DMAB solution (Fujitani N, Sakai S, Yamaguchi Y, Takenaka H, "Inhibitory effects of microalgae on the activation of hyaluronidase", J. Appl. Phycol., 13, 489-492 (2001))

The p-DMAB solution was prepared by diluting 10 × stock solution (5 g of p-dimethylaminobenzaldehyde, 6 ml of 10 M HCl, 44 ml of acetic acid) with acetic acid immediately prior to use.

2. Test results

Table 3 shows the obtained results of the inhibition on a hyaluronidase activity.

[Table 3]

| Hyaluronidase activity inhibition of exopolysaccharides produced by the strain IJH-SONE68 | |
| --- | --- |
| Tested samples | IC$_{50}$ ($\mu$g/ml) |
| Polysaccharide sample of IJH-SONE68 (containing neutral and acidic polysaccharide fractions) | 370 |
| Neutral polysaccharide fractions of IJH-SONE68 | 550 |
| Acidic polysaccharide fractions of IJH-SONE68 | 1200 |
| Fucoidan (Laminaria Japonic) | 2000<* |
| Ketotifen fumarate | 2000<* |
| Dipotassium glycyrrhizinate | 530 |
| * Hyaluronidase activity inhibition was not observed until the concentration of 2000 $\mu$g/ml | |

As is clear from the results in Table 3, the polysaccharide sample (containing the neutral polysaccharide fractions and acidic polysaccharide fractions), the neutral polysaccharide fractions and acidic polysaccharide fractions, which were exopolysaccharides produced by the strain IJH-SONE68, exhibited a high hyaluronidase inhibitory activity. In particular, the polysaccharide sample and the neutral polysaccharide fractions exhibited a hyaluronidase inhibitory activity comparable to that of dipotassium glycyrrhizinate having anti-inflammatory action.

As is clear from the foregoing detailed descriptions, the present invention provides the following inventions:

[1] An exopolysaccharide of a lactic acid bacterium derived from a fig and belonging to *Lactobacillus paracasei;*

[2] The exopolysaccharide according to the above [1], which is a neutral polysaccharide having a structure in which N-acetylglucosamines are linked with each other via $\alpha$-1,6 bond;

[3] The exopolysaccharide according to the above [1], which is an acidic polysaccharide mainly composed of glucoses and mannoses;

[4] The exopolysaccharide according to any one of the above [1] to [3], which has a hyaluronidase inhibitory activity;

[5] The exopolysaccharide according to any one of the above [1] to [4], wherein the lactic acid bacterium is *Lactobacillus paracasei* strain IJH-SONE68 (Accession No. NITE BP-02242) or a lactic acid bacterium equivalent thereto;

[6] The exopolysaccharide according to any one of the above [1] to [5], obtainable by isolating and purifying, by ion exchange chromatography, polysaccharides obtained from the culture of a lactic acid bacterium derived from a fig and belonging to *Lactobacillus paracasei;*

[7] The exopolysaccharide according to any one of the above [1] to [6], obtainable by the steps of (1) removing bacterial cell bodies by centrifugation from the culture of a lactic acid bacterium derived from a fig and belonging to *Lactobacillus paracasei*; (2) recovering polysaccharides and proteins as precipitates from the culture obtained in the step (1), by precipitation with ethanol or acetone; (3) removing the proteins from the recovered precipitates to recover the exopolysaccharides; and (4) isolating and purifying the recovered exopolysaccharides by anion exchange chromatography;

[8] A composition comprising the exopolysaccharide according to any one of the above [1] to [7];

[9] The composition according to the above [8], which is a food and drink composition;

[10] The composition according to the above [9], wherein the food and drink are a functional food, a fermented food, a beverage or a supplement;

[11] The composition according to the above [8], which is a pharmaceutical composition;

[12] The composition according to the above [8], which is a feed composition;

[13] The composition according to the above [8], which is a cosmetic composition;

[14] The composition according to any one of the above [8] to [13], which is for a hyaluronidase inhibition;

[15] The composition according to any one of the above [8] to [13], which is for an antiallergy;

[16] A method for preparing the exopolysaccharide according to any one of the above [1] to [7], which comprising obtaining polysaccharides from the culture of a lactic acid bacterium derived from a fig and belonging to *Lactobacillus paracasei*, and isolating and purifying the obtained polysaccharides by an ion exchange chromatography;

[17] The method according to the above [16], which comprises the steps of (1) removing bacterial cells by centrifugation from the culture of a lactic acid bacterium derived from a fig and belonging to *Lactobacillus paracasei*; (2) recovering polysaccharides and proteins as precipitates from the culture obtained in the step (1), by precipitation due to ethanol or acetone; (3) removing the proteins from the recovered precipitates to recover the exopolysaccharides; and (4) isolating and purifying the recovered exopolysaccharides by anion exchange chromatography;

[18] Use of the exopolysaccharide according to any one of the above [1] to [7] as an active ingredient of a composition;

[19] The use according to the above [18], wherein the composition is a food and drink composition;

[20] The use according to the above [19], wherein the food and drink are a functional food, a fermented food, a beverage or a supplement;

[21] The use according to the above [18], wherein the composition is a pharmaceutical composition;

[22] The use according to the above [18], wherein the composition is a feed composition;

[23] The use according to the above [18], wherein the composition is a cosmetic composition;

[24] The use according to any one of the above [16] to [21], wherein the composition is for a hyaluronidase inhibition;

[25] The use according to any one of the above [16] to [21], wherein the composition for an antiallergy;

[26] A method for preparing a composition, comprising mixing the exopolysaccharide according to any one of the above [1] to [7] with other component;

[27] The preparation method according to the above [26], wherein the composition is a food and drink composition;

[28] The preparation method according to the above [27], wherein the food and drink are a beverage, a functional food, a fermented food or a supplement;

[29] The preparation method according to the above [26], wherein the composition is a pharmaceutical composition;

[30] The preparation method according to the above [26], wherein the composition is a feed composition;

[31] The preparation method according to the above [26], wherein the composition is a cosmetic composition;

[32] The preparation method according to any one of the above [26] to [31], wherein the composition is for a hyaluronidase inhibition;

[33] The preparation method according to any one of the above [26] to [31], wherein the composition is for an antiallergy;

[34] A preparation method for applying, to a subject in need thereof, the exopolysaccharide according to any one of the above [1] to [7] which comprises applying a composition comprising the exopolysaccharide according to any one of the above [1] to [7] to the subject;

[35] The application method according to the above [34], wherein the composition is a food and drink composition;

[36] The application method according to the above [35], wherein the food and drink are a beverage, a functional food, a fermented food or a supplement;

[37] The application method according to the above [34], wherein the composition is a pharmaceutical composition;

[38] The application method according to the above [34], wherein the composition is a feed composition;

[39] The application method according to the above [34], wherein the composition is a cosmetic composition;

[40] The application method according to one of any one of the above [34] to [39], wherein the composition exerts a hyaluronidase inhibition action on the subject; and

[41] The application method according to one of any one of the above [34] to [39], wherein the composition exerts an antiallergy action on the subject.

Industrial Applicability

As described in detail herein above, the exopolysaccharide of the present invention exerts a hyaluronidase inhibitory activity and exhibits antiallergy effect. Therefore, the exopolysaccharide of the present invention can be used as an active ingredient of a food and drink, a medicine, a feed, a cosmetic and the like.

**Claims**

1.  An exopolysaccharide of a lactic acid bacterium derived from a fig and belonging to *Lactobacillus paracasei.*

2.  The exopolysaccharide according to claim 1, which is a neutral polysaccharide having a structure in which N-acetylglucosamines are linked with each other via $\alpha$-1,6 bond.

3.  The exopolysaccharide according to claim 1, which is an acidic polysaccharide mainly composed of glucose and mannose.

4.  The exopolysaccharide according to any one of claims 1 to 3, which has a hyaluronidase inhibitory activity.

5.  The exopolysaccharide according to any one of claims 1 to 4, wherein the lactic acid bacterium is *Lactobacillus paracasei* strain IJH-SONE68 (Accession No. NITE BP-02242) or a lactic acid bacterium equivalent thereto.

6.  The exopolysaccharide according to any one of claims 1 to 5, obtainable by isolating and purifying, by ion exchange chromatography, polysaccharides obtained from the culture of a lactic acid bacterium derived from a fig and belonging to *Lactobacillus paracasei.*

7.  The exopolysaccharide according to any one of claims 1 to 6, obtainable by the steps of (1) removing bacterial cells by centrifugation from the culture of a lactic acid bacterium derived from a fig and belonging to *Lactobacillus paracasei;* (2) recovering polysaccharides and proteins as precipitates from the culture obtained in the step (1), by precipitation with ethanol or acetone; (3) removing the proteins from the recovered precipitates to recover the exopolysaccharides; and (4) isolating and purifying the recovered exopolysaccharides by anion exchange chromatography.

8.  A composition comprising the exopolysaccharide according to any one of claims 1 to 7.

9.  The composition according to claim 8, which is a food and drink composition.

10. The composition according to claim 9, wherein the food and drink are a functional food, a fermented food, a beverage, or a supplement.

11. The composition according to claim 8, which is a pharmaceutical composition.

12. The composition according to claim 8, which is a feed composition.

13. The composition according to claim 8, which is a cosmetic composition.

14. The composition according to any one of claims 8 to 13, which is for a hyaluronidase inhibition.

15. The composition according to claim 14, which is for an antiallergy.

FIG.1

(A)

(B)

EP 3 483 281 A1

## FIG.2

FIG.3

（A）

Proton-NMR

（B）

Carbon-NMR

## FIG.4 Structural analysis of neutral exopolysaccharide

NMR measurement (Bruker Avance- III, 600MHz)
Measurement solvent $D_2O$
Measurement time 60 hours
Measurement items
One dimensional NMR (H, C ,DEPT)
Two-dimensional NMR (HSQC, HMBC (indicated by arrows), COSY (indicated by bold lines))

3.69(dd, 5.9, 10.5 Hz)
4.03(brd, 10.5 Hz)
69.1

3.38(brd, 10.5 Hz)
75.5

4.68(brs)
100.1

3.35(brd, 9.8 Hz)
67.1

4.42(d, 3.5 Hz)
52.9

1.95(s)
22.3

3.66(d, 3.5, 9.8 Hz)
71.8

175.5

Two-dimensional NMR

Numerical values indicate chemical shift values.
Underlined numerical values indicate carbon-NMR values, and non-underlined numerical values indicate proton-NMR values.
 Neutral exopolysaccharide is shown to have a structure in which $\alpha$-N-acetylglucosamines range in the manner that
1-position of $\alpha$-N-acetylglucosamine binds to 6-position of other $\alpha$-N-acetylglucosamine.

# FIG.5

pce1A B C D E F G H I J K L M N O P Q R

1.0 kb

pce1 cluster
(23 kb)

Lb. paracasei IJH-SONE68
Genome DNA

2,100,000

34 kb

2,200,000

pce2 cluster
(28 kb)

C
|
Truncated

D E F G H I J K L M N O O'

短縮Truncated

Truncated

1.0 kb

▶ Priming glycosyltransferase    ▶ Glycosyltransferase    ▷ Polymerase    ▷ Flippase    ▷ Chain length-regulator

▷ Modifying enzyme    ▷ Unknown    ▶ Transposase    ▷ Non-related gene

FIG.6

(A)

ATCC 334

IJH-SONE68

JCM 8130$^T$

pce1 cluster   pce2 cluster

3,000,000   2,500,000   2,000,000   1,500,000   1,000,000   500,000   0   [bp]

(B)

1.0 kb

IJH-SONE68

pce1A B C D E F G H I J K L M N O P Q R

JCM 8130$^T$

lbpc_1936 37 38 39 40 41 42 43 44 45 46 47 48 49 50 51 52 53 54 55 56 57 58

**INTERNATIONAL SEARCH REPORT**

| International application No. |
| --- |
| PCT/JP2018/020334 |

| A. CLASSIFICATION OF SUBJECT MATTER |
| --- |
| Int.Cl. C12P19/04(2006.01)i, A23K20/163(2016.01)i, A23L33/125(2016.01)i, A61K8/73(2006.01)i, A61K31/715(2006.01)i, A61P37/08(2006.01)i, C08B37/00(2006.01)i, C12P19/28(2006.01)i, B01D15/36(2006.01)n, C12N1/20(2006.01)n |

According to International Patent Classification (IPC) or to both national classification and IPC

| B. FIELDS SEARCHED |
| --- |
| Minimum documentation searched (classification system followed by classification symbols) |
| Int.Cl. C12P19/04, A23K20/163, A23L33/125, A61K8/73, A61K31/715, A61P37/08, C08B37/00, C12P19/28, B01D15/36, C12N1/20 |

| Documentation searched other than minimum documentation to the extent that such documents are included in the fields searched | |
| --- | --- |
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

| Electronic data base consulted during the international search (name of data base and, where practicable, search terms used) |
| --- |
| JSTPlus/JMEDPlus/JST7580 (JDreamIII), CAplus/REGISTRY (STN), MEDLINE/EMBASE/BIOSIS/WPIDS/WPIX (STN), GenBank/EMBL/DDBJ/GeneSeq |

| C. DOCUMENTS CONSIDERED TO BE RELEVANT | | |
| --- | --- | --- |
| Category* | Citation of document, with indication, where appropriate, of the relevant passages | Relevant to claim No. |
| X Y | PANTHAVEE, W. et al., "Characterization of exopolysaccharides produced by thermophilic lactic acid bacteria isolated from tropical fruits of Thailand", Biol. Pharm. Bull. (2017) vol. 40, pp. 621–629, page 621, abstract, page 626, table 4, page 622, left column, last paragraph to right column, paragraph [0002] | 1, 3–7 1–15 |

☒ Further documents are listed in the continuation of Box C.     ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 07 August 2018 (07.08.2018) | 14 August 2018 (14.08.2018) |

| Name and mailing address of the ISA/ Japan Patent Office 3-4-3, Kasumigaseki, Chiyoda-ku, Tokyo 100-8915, Japan | Authorized officer |
| --- | --- |
| | Telephone No. |

Form PCT/ISA/210 (second sheet) (January 2015)

**INTERNATIONAL SEARCH REPORT**

| International application No. |
| --- |
| PCT/JP2018/020334 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X<br>Y | KANG, H. et al., "Exopolysaccharide-overproducing Lactobacillus paracasei KB28 induces cytokines in mouse peritoneal macrophages via modulation of NF-κB and MAPKs", J. Microbiol. Biotechnol. (2011) vol. 21, no. 11, pp. 1174-1178, page 1174, abstract, page 1176, fig. 1 | 1, 3-7<br>1-15 |
| Y | Database DDBJ/EMBL/GenBank [online], Accession No. CP007122. 1,<br><https://www.ncbi.nlm.nih.gov/nuccore/CP007122.1?from=1960345&to=1961923&report=gbwithparts&strand=2>, 01 October 2014, uploaded [retrieved on 20 July 2018], WANG, S., et al., Definition: Lactobacillus paracasei N1115, complete genome. "DEFINITION", "FEATURES", "ORIGIN" | 1-15 |
| Y | JP 2011-201781 A (MORISHITA JINTAN CO., LTD.) 13 October 2011, claims, paragraphs [0032], [0034], [0048]-[0049] & US 2013/0005959 A1, claims, paragraphs [0039], [0041], [0060]-[0061] & EP 2551283 A1 & WO 2011/118552 A1 | 6-15 |
| Y | JP 2001-520035 A (SOCIETE DES PRODUITS NESTLE S.A.) 30 October 2001, claims & US 2004/0023361 A1, claims & EP 1023435 A1 & WO 1999/020739 A2 | 8-15 |
| P, X | NODA, M. et al., "A novel structure of exopolysaccharide produced by a plant-derived lactic acid bacterium Lactobacillus paracasei IJH-SONE68", J. Biochem. (2018) vol. 164, no. 2, pp. 87-92, page 87, abstract, page 87, right column, paragraph [0004], page 88, fig. 1, page 89, fig. 2, page 90, table 3 | 1-15 |

Form PCT/ISA/210 (continuation of second sheet) (January 2015)

## REFERENCES CITED IN THE DESCRIPTION

**Patent documents cited in the description**

- WO 2009131208 A
- WO 2012133827 A
- JP 2016123382 A
- JP 2007189973 A
- JP 2016113378 A
- JP 2016037451 A
- JP 2011142907 A

**Non-patent literature cited in the description**

- *Appl. Microbiol. Biotechnol.,* 2009, vol. 84, 341-347
- *Int. J. Food Microbiol.,* 2007, vol. 113, 358-361
- *PLoS One,* 2012, e30696
- *Biosci. Biotechnol. Biochem.,* vol. 73, 1561-1565
- *Carbohydr. Polym.,* 2015, vol. 124, 292-301
- *Biol. Pharm. Bull.,* 1994, vol. 17, 1012-1017
- *Appl. Environ. Microbiol.,* 2017, vol. 83, e02702-16
- *J. Microbiol. Biotechnol.,* 2011, vol. 21, 1174-1178
- *Carbohdr. Res.,* 1996, vol. 285, 129-139
- *Genes Dev.,* March 1993, vol. 7 (3), 468-478
- *J. Biol. Chem.,* 29 January 1999, vol. 274 (5), 3215-3221
- 16S/23S rRNA sequencing. **LANE, DJ.** Nucleic Acid Techniques in Bacterial Systematics. Wiley, 1991, 115-175
- *Nat. Methods,* vol. 10, 563-56933
- **KIMMEL SA ; ROBERTS RF.** Development of a growth medium suitable for exopolysaccharide production by Lactobacillus delbrueckii ssp. Bulgaricus RR. *Int. J. Food Microbiol.,* 1998, vol. 40, 87-92
- **KETS EPW ; GALINSKI EA ; DE BONT JAM.** Carnitine: ''A novel compatible solute in Lactobacillus plantarum. *Arch. Microbiol.,* 1994, vol. 192, 243-248
- **DUBOIS M ; GILLES KA ; HAMILTON JK ; REBERS PA ; SMITH F.** Colorimetric method for determination of sugars and related substances. *Anal. Chem.,* 1956, vol. 28, 350-356
- **YOTHER J.** *Annu. Rev. Microbiol.,* 2011, vol. 65, 563-581
- **VAN KRANENBURG R ; VOS HR ; VAN SWAM II ; KLEEBEZEM M ; DE VOS WM.** *J. Bacteriol.,* October 1999, vol. 181 (20), 6347-6353
- **MAKAROVA, K.** *Proc. Natl. Acad. Sci. U.S.A.,* 2006, vol. 103 (42), 15611-15616
- **TOH, H.** *PLoS ONE,* 2013, vol. 8, e75073
- **FUJITANI N ; SAKAI S ; YAMAGUCHI Y ; TAKENAKA H.** Inhibitory effects of microalgae on the activation of hyaluronidase. *J. Appl. Phycol.,* 2001, vol. 13, 489-492